# EUROPEAN PATENT SPECIFICATION

(11) **EP 0 499 206 B1**
(45) Date of publication and mention of the grant of the patent: **02.07.1997**
(21) Application number: 92102265.3
(22) Date of filing: 11.02.1992
(51) Int. Cl.: C08G 65/32, C07C 229/18

(54) **Polyamine compositions containing secondary amine functions**
Polyaminzusammensetzungen, die sekundäre Aminogruppen enthalten
Compositions des polyamines qui contiennent des groupes amines secondaires

(30) Priority: 12.02.1991 JP 41324/91; 30.04.1991 JP 128417/91; 30.04.1991 JP 128418/91
(43) Date of publication of application: 19.08.1992
(73) Proprietor: NIPPON PAINT CO., LTD., Osaka-shi Osaka 572 (JP)
(72) Inventor: Ohshima, Toshiyuki, Ibaraki-shi, Osaka-fu (JP); Ishibashi, Hideo, Neyagawa-shi, Osaka-fu (JP); Tamura, Rie, Toyonaka-shi, Osaka-fu (JP); Yamamoto, Satoshi, Hirakata-shi, Osaka-fu (JP); Izumo, Takaharu, Mishima-gun, Osaka-fu (JP)
(74) Representative: Kinzebach, Werner, Dr.

(56) References cited:
- EP-A- 0 046 088
- EP-A- 0 103 211
- US-A- 5 075 503
- DATABASE WPIL Week 9046, Derwent Publications Ltd., London, GB; AN 90-346091
- DATABASE WPIL Week 8402, Derwent Publications Ltd., London, GB; AN 84-008043

## Description

### BACKGROUND OF THE INVENTION

This invention relates to a novel polyamine composition for use as a raw material in the manufacture of plastics such as in the reaction injection molding (RIM) of polyurea.

Polyoxyalkylene polyamines find use as a raw material in the manufacture of polyurea elastomers and RIM as well as in hardening epoxy resins. It has been known that when polyoxyalkylene polyamines are modified to have a plurality of secondary amino groups at the terminal ends of the molecule, the resulting products will exhibit excellent reactivity with polyisocyanate compounds compared with the corresponding primary polyoxyalkylene polyamines and, therefore, the resulting polyurea products may have excellent properties.

Aromatic diamines and xylylene diamines also find use in the polyurea RIM and the like. The modification of their primary amino groups to secondary amino groups also result in benefical effects on the reactivity with polyisocyanates as well as properties of the resulting polyurea products.

One of prior art methods for producing secondary amine-terminated polyoxyalkylene polyamines is disclosed in Japanese Laid Open Patent Application (Kokai) Nos. 38425/1990 and 127425/1989. This method includes hydrogenolytic aminolysis of the corresponding polyoxyalkylene polyols with a primary amine. This method suffers from certain disadvantages in that the reaction takes place only under high pressure and high temperature in an autoclave in the presence of a catalyst such as Raney nickel. Another known method is disclosed in Japanese Laid Open Patent Application (Kokai) No. 153931/1990 and includes alkylation of the corresponding primary polyamines with alkyl halides. This method also suffers from certatin disavantages in that a portion of the starting primary amine is necessarily comsumed for binding the hydrogen halide by-product or an excess of acid-binding agent such as alkali metal hydroxides must be added. Besides, the conversion rate to secondary amino groups is relatively low and the alkylating agent is relatively expensive.

The alkylation method may also find its application to the production of secondary aromatic diamines and xylylenediamines from the corresponding primary amines as disclosed, for example, in Japanese Laid Open Patent Application (Kokai) Nos.311116/1990 and 251515/1990, respectively. In addition to various deficiencies as discussed above, alkylation of primary diamines of this type proceeds stepwise and one of primary amino groups may be alkylated only with difficulty once the other primary amino group has been alkylated. Accordingly, this method is utilized in practice for the production of asymmetric diamines in which one amino group is primary and the other is secondary.

All of the above discussed prior art methods generally give a secondary amine of which second hydrocarbon substituent is an alkyl or aralkyl radical free of a functioinal group. In contrast with this, Japanese Laid Open Patent Application (Kokai) No.251515/1990 discloses the Michael reaction of a polyamine with an acrylic or methacrylic monomer to convert low molecular weight, straight chain, aliphatic primary diamines or alicyclic primary diamines to the corresponding secondary diamines. The reaction products have a secondary amino group of which second hydrocarbon substituent has a functional group originating from the acrylic or methacrylic monomer. In our experiments, however,it has been revealed that the secondary amine content of the products of this method is unsatisfactorily low particularly when the monomer is a methacrylic monomer.

Further, EP 0 046 088 describes the Michael reaction of polyoxyalkylene polyamines with derivatives of acrylic acid such as hydroxy ethyl acrylate, methacrylate or α-cyanoacrylate and diethylene glycol monoacrylate,or with compounds having oxirane rings such as ethylene oxide, propylene oxide, n-butylglycidyl ether and styrene oxide. The reaction products can be advantageously used as curing agent for polyurethane preparation.

Accordingly, a need exists for a secondary polyamine composition and the method of making the same which eliminate or ameliorate deficiencies of the prior art compositions and methods.

### SUMMARY OF THE INVENTION

The present invention provides a composition for use in the manufacture of plastics comprising a polyamine product obtainable by reacting an acrylate ester selected from n-butyl acrylate, isobutyl acrylate, t-butyl acrylate and 2-ethylhexyl acrylate, with a polyamine selected from a polyoxypropylene triamine, tolylenediamine and diethyltolylenediamine, to convert the primary amino groups of said polyamine at least in part to secondary amino groups of the formula:

- NH - CH₂ - CH₂ - COOR

wherein R is a n-butyl, isobutyl, t-butyl, or 2-ethylhexyl group.

### DETAILED DISCUSSION

The production of the polyamine composition of this invention involves the following Michael reaction.

-NH₂+CH₂=CH-COOR→-NH-CH₂-CH₂-COOR

Accordingly, the secondary polyamine formed by this reaction is different in structure from known secondary amines in that the second hydrocarbon substituent of the former contains an electron-withdrawing group.

Starting primary polyamines used in the present invention include polyoxypropylene triamines, tolylenediamines and diethyltolylenediamines which are conventionally used in the production of polyurea elastomers and RIM.

The Polyoxypropyl triamines having a plurality of primary amino groups at the terminals of the molecule may be produced as disclosed, for example, in Belgian Patent No.677124 by the hydrogenolytic ammonolysis of the corresponding polyoxypropylene polyols. The polyoxypropylene polyols may be produced, in turn, by addition polymerizing an alkylene oxide such as ethylene oxide, propylene oxide, butylene oxide, tetrahydrofuran or a mixture thereof using an initiator in the presence of a basic catalyst such as alkali metal hydroxides. Usable initiators include water; polyols such as ethylene glycol, di-, tri- or other polyethylene glycols, propylene glycol, di-, tri- or other polypropylene glycols, glycerine, diglycerine, pentaerythritol, sorbital and sucrose; polyphenols such as bisphenol A, bisphenol S and resorcin; and alkanolamines such as diethanolamine and triethanolaine. Polyoxypropylene polyols having an average molecular weight from 200 to 10,000, preferable from 400 to 8,000 may be used.

The polyoxypropylene triamines are commercially available including polyoxypropylene triamines sold under the name of Texrim TR-5050 (Texaco Chemical, amine equivalent about 1930) and Jeffamine T-403 (Texaco Chemical, amine equivalent about 160). These commercial products may advantageously be used in the present invention.

Aromatic diamines usable in the present invention include tolylenediamines or diethyltolylenediamines such as 2,4- or 2,6-diaminotoluene, 2,4- or 2,6-diamino-1-methyl-3,5-diethylbenzene.

A variety of aromatic diamines are commercially available including those sold under the name of Etacure 100 (Asano Chemicals, a mixture of 1-methyl-3,5-diethyl-2,4-diaminobenzene and 1-methyl-3,5-diethyl-2,6-diaminobenzene) and Tolylenediamine (Mitsui Toatsu Chemicals, 2, 4-diaminotoluene).

These commercial products may advantageously used in the present invention.

As stated before, the present invention utilizes as ethylenically unsaturated compound or monomer an acrylate ester selected from n-butyl acrylate, isobutyl acrylate, t-butyl acrylate and 2-ethylhexyl acrylate, in the Michael reaction with the primary polyamines.

We have discovered that the Michael reaction of the primary polyamine and the ethylenic monomer may unexpectedly be promoted by the presence of an acidic or neutral esterification catalyst.

Examples of usable catalysts include inorganic or organic acid such as hydrochloric, sufuric, nitric, phosphoric, propionic, formic, acetic, dichloroacetic, trichloroacetic, trifluoroacetic, benzoic or p-toluenesulfonic acid; unitary metal oxides such as aluminum oxide, silicon oxide and niobium oxide; complexed metal oxides such as SiO₂/Al₂O₃, TiO₂/SiO₂, SiO₂/La₂O₃ and TiO₂/Al₂O₃; sulfides such as zinc sulfide; sulfate such as nickel sulfate and copper sulfate; phosphates such as aluminum phosphate and titanium phosphate; chloride such as aluminum chloride and copper chloride; clays such as acid clay, montmorillonite and kaolin; solidified acids such as solidified phosphoric acid, solidified sulfuric acid and solidified boric acid; and acidic ion exchange resins. Also included are organotin compounds such as dibutyltin oxide and dibutyltin dilaurate; organoaluminum compounds such as aluminum isopropylate, mono-sec.-butoxyaluminum diisopropylate, aluminum ethylate, aluminum ethylacetoacetate diisopropylate, aluminum tris-ethylacetoacetate and aluminum bis-ethylacetoacetate monoacetylacetonate; organotitanium compounds such as tetraisopropoxytitanium, tetra-n-butoxytitanium, tetrakis-2-ethylhexoxytitanium, tetrakis-stearyloxy-titanium, diisopropoxy-bis(acetylacetonato)titanium, di-n-butoxy-bis(tri-ethanol-ammine)titanium and hydroxy-bis(lactato)titanium.

We have also discovered that the Michael reaction of the primary polyoxypropylene triamine and the ethylenic monomer may be promoted by the presence of a benzenoid compound having at least one electron-donating or electron-withdrawing substituent on the benzenoid ring in place of or in addition to the esterification catalyst. Quinones are also useful for promoting the Michael reaction. Examples of useful benzenoid compounds and quinones include alkyl or aralkyl-substituted benzenoids such as toluene, xylene, ethylbenzene, t-butylbenzene and diphenylmethane; aromatic amines such as aniline, N,N-dimethylaniline, diaminotoluene, xylidine, diaminodiphenylmethane, bis-(N-ethylamino)toluene and aminonaphthalene; hydroxyl compounds such as phenol, cresol and naphthol; halo compounds such as fluorobenzene, chlorobenzene, bromobenzene, iodobenzene, chloronaphthalene and iodonaphthalene; nitro compounds such as nitrobenzene, dinitrotoluene and dinitronaphthalene; cyano compounds such as benzonitrile and naphthonitrile; ketones such as acetophenone and propiophenone; benzenoid compounds having a combination of mentioned substituents; and quinones such as benzoquinone and naphthoquinone.

The ratio of the ethylenic monomer relative to the starting polyamine may vary within a wide range as desired. This ratio in terms of equivalent of ethylenic function relative to the primary amine function may range from 0.01 to 100, preferably from 1 to 10. This means that the presence of an amount of unreacted primary amine functions or ethylenic functions may be tolerated in the reaction mixture.

The amount of the esterification catalysts may vary, when used, but should be no more than the catalytically effective amount.

The amount of aromatic compounds having an electron-donating or withdrawing group should be no more than the tolerable limit in the modified polyoxypropylene triamine composition. This amount is generally no more than one equivalent relative to the primary amine function of the starting polyoxypropylene triamine.

The Michael reaction may be performed at a temperature from room temperature to about 150°C. After the reaction, the reaction mixture may be used as such as a raw material in the polyurea RIM. If the reaction mixture includes an amount of unreacted ethylenic monomer and/or catalyst, these impurities may be removed by polymerizing, neutralizing or other suitable means.

The following examples are intended to further illustrate the present invention. All parts and percents therein are by weight unless otherwise indicated.

### Example 1

A 500ml flask equipped with a stirrer, reflux condenser, drip funnel, thermometer and nitrogen gas tube was charged with 386g of Texrim TR-5050(trifunctional polyoxyalkylene polyamine, amine equivalent 1930, average MW 5000) and heated to 120°C. To this was added 73.6g of 2-ethylhexyl acrylate dropwise over 4 hours and allowed to react for additional 24 hours. The conversion rate of the reaction mixture into secondary amine was measured by the salicylaldehyde method according to the modified Wagner method (C.D.Wagner et al., J.Am.Chem.Soc.,69, 2609-2611(1947). The result is shown in Table 1. The conversion rate represent percent of secondary amine number relative to the total amine number.

### Example 2

To the same reactor as used in Example 1 were placed 386g of Texrim TR-5050 and 19.8g of diaminodiphenylmethane. Then 73.6g of 2-ethylhexyl acrylate was added dropwise over 4 hours and allowed to react for additional 36 hours. The conversion rate is shown in Table 1.

### Example 3

Example 2 was repeated except that 18.6g of aniline was replaced for diaminodiphenylmethane. The conversion rate is shown in Table 1.

### Example 4

Example 2 was repeated except that 21.2g of ethylbenzene was replaced for diaminodiphenylmethane. The conversion rarte is shown in Table 1.

### Example 5

Example 2 was repeated except that 51.2g of n-butyl acrylate was replaced for 2-ethylhexyl acrylate and the reaction was continued for 20 hours. The conversion rate is shown in Table 1.

### Example 6

To the same reactor as used in Example 1 was placed 200g of Jeffamine T-403 (trifunctional polyoxypropylene polyamine, amine equivalent 160, average MW 400). To this was added 230g of 2-ethylhexyl acrylate dropwise over 2 hours and allowed to react for additional 3 hours. The conversion rate is shown in Table 1.

### Example 7

The same reactor as used in Example 1 was charged with 386g of Texrim TR-5050 and 0.6g of acetic acid, and heated to 120°C. To this was added 73.6g 2-ethylhexyl acrylate dropwise over 4 hours and allowed to react for additional 4 hours. The conversion rate is shown in Table 2.

### Example 8

Example 7 was repeated except that 1.9g of p-toluenesulfonic acid was replaced for acetic acid. The conversion rate is shown in Table 2.

### Example 9

Example 8 was repeated except that 36.8g of 2-ethylhexyl acrylate was added dropwise over 2 hours and allowed to react for additional 6 hours. The conversion rate is shown in Table 2.

### Example 10

Example 8 was repeated except that 51.2g of n-butyl acrylate was replaced for 2-ethylhexyl acrylate. The conversion rate is shown in Table 2.

### Example 11

Example 7 was repeated except that 20g of solidified phosphoric acid E48A1 (P₂O₅/SiO₂/TiO₂ type sold by JGC Corporation) was replaced for acetic acid. The conversion rate is shown in Table 2.

### Example 12

Exampel 7 was repeated except that 20g of silicaalumina catalyst N631HN (JGC Corporation) was replaced for acetic acid. The conversion rate is shown in Table 2.

### Example 13

Example 7 was repeated except that 3.4g of tetrabutoxytitanium was replaced for acetic acid. The conversion rate is shown in Table 2.

### Example 14

Example 7 was repeated except that 6.3g of dibutyltin dilaurate was replaced for acetic acid. The conversion rate is shown in Table 2.

### Example 15

The same reactor used in Example 1 was charged with 200g of Jeffamine T-403 and 11.9g of p-toluenesulfonic acid, and heated to 80°C. To this was added 230g of 2-ethylhexyl acrylate dropwise over 2 hours and allowed to react for additional 3 hours. The conversion rate is shown in Table 2.

### Example 16

A 500ml flask equipped with a stirrer, reflux condenser, drop funnel, thermometer and nitrogen gas tube was charged with 91.5g of tolylenediamine (bifunctional, amine equivalent 61, MW 122) and 14.3g of p-toluenesulfonic acid, and heated to 120°C. To this was added 192g of n-butyl acrylate dropwise over 2 hours and allowed to react for additional 12 hours. The product was tested for the formation and per cent conversion into secondary amine by the FT-IR spectrophotometry, liquid chromatography and ¹³C-NMR. The conversion rate is shown in Table 3.

### Example 17

Example 16 was repeated except that 276g of 2-ethylhexyl acrylate was replaced for n-butyl acrylate. The conversion rate is shown in Table 3.

### Example 18

As in Example 16, 133.5g of Etacure 100 (diethyltolylenediamine, amine equivalent 89, MW 178 sold by Asano Chemicals Co.,Ltd.) was reacted with 192g of n-butyl acrylate for 24 hours. The conversion rate is shown in Table 3.

### Example 19

Example 16 was repeated except that 4.5g of acetic acid was replaced for p-toluenesulfonic acid. The conversion rate is shown in Table 3.

### Example 20

Example 16 was repeated except that 20g of solidified phosphoric acid E48A1 was replaced for p-toluen -sulfonic acid. The conversion rate is shown in Table 3.

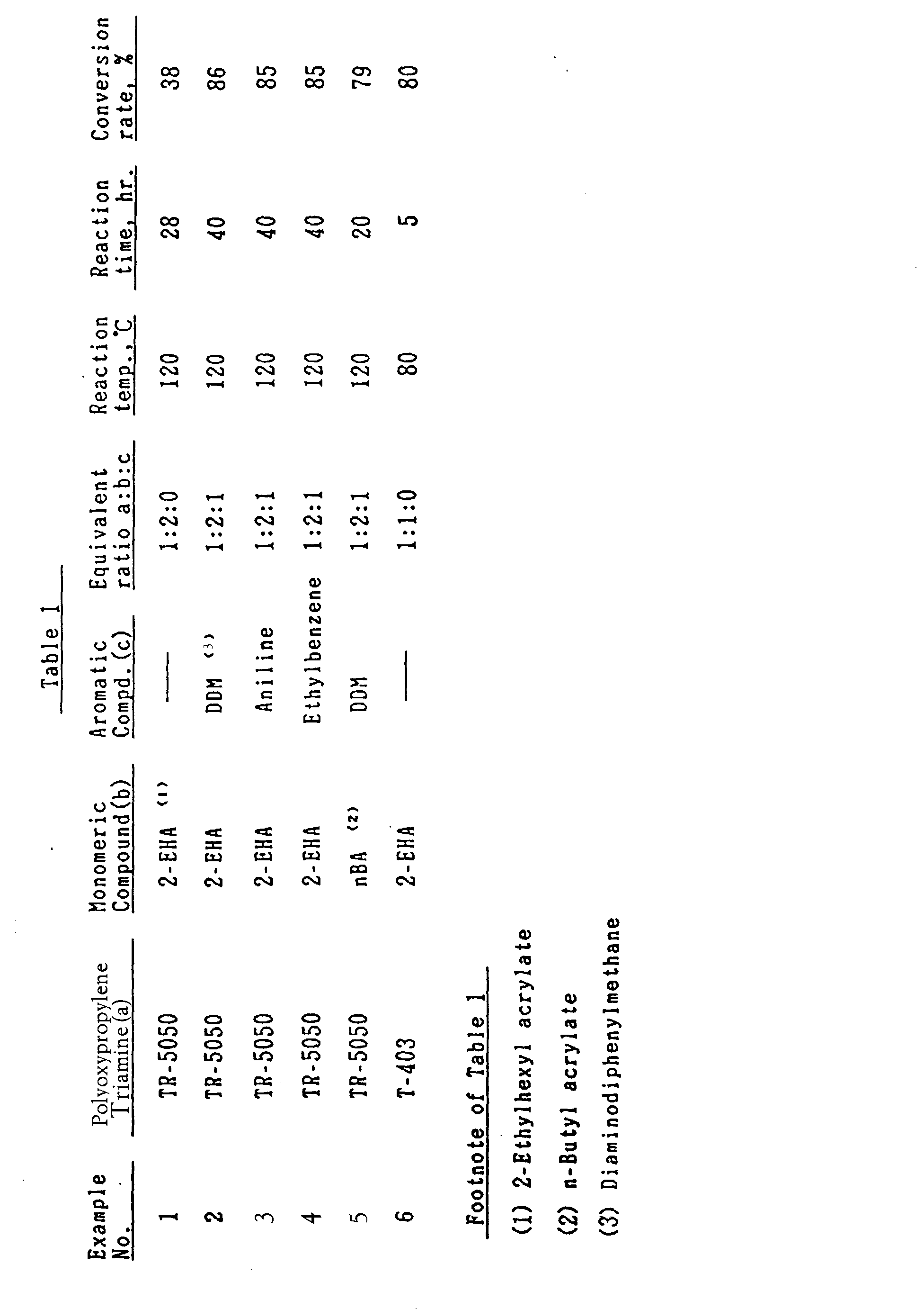

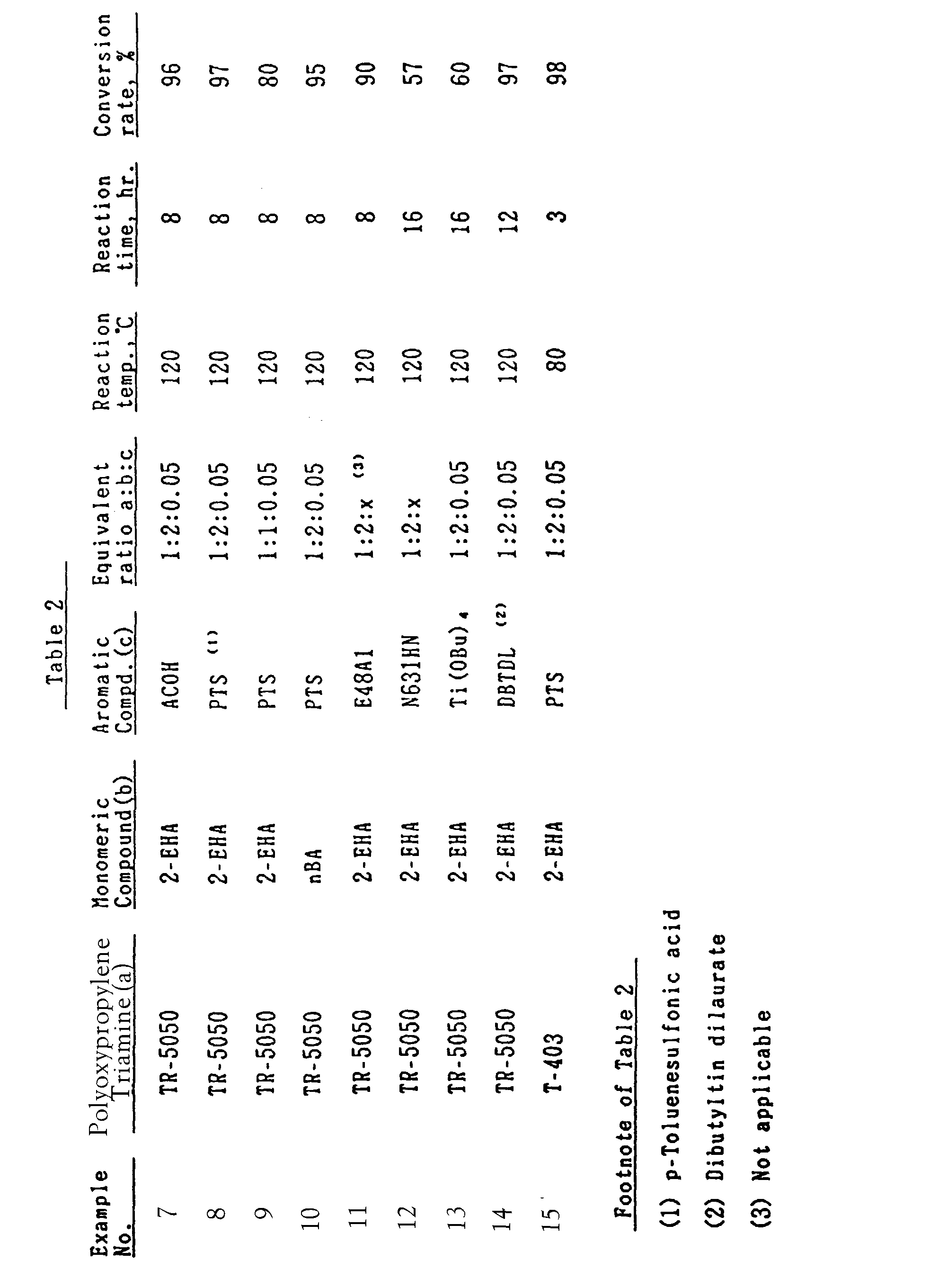

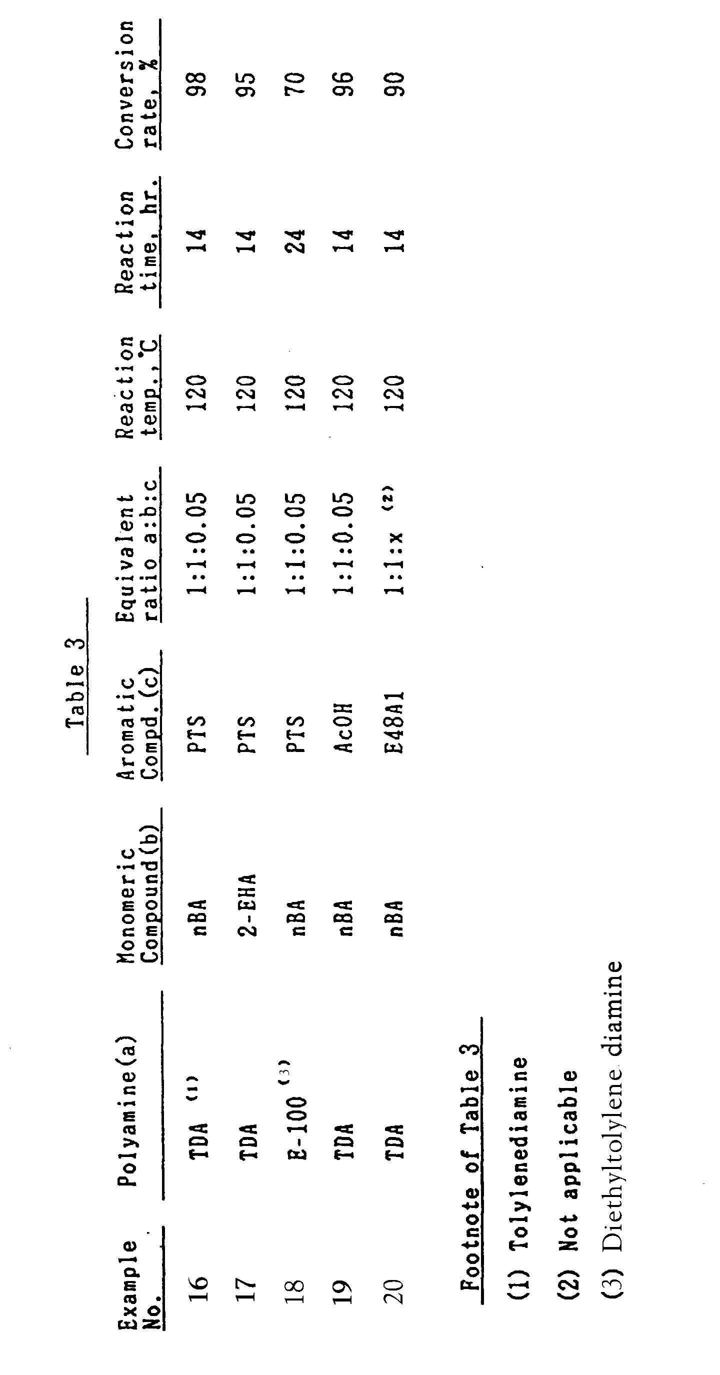

## Claims

1. A composition for use in the manufacture of plastics comprising a polyamine product obtainable by reacting an acrylate ester selected from n-butyl acrylate, isobutyl acrylate, t-butyl acrylate and 2-ethylhexyl acrylate, with a polyamine selected from a polyoxypropylene triamine, tolylenediamine and diethyltolylenediamine, to convert the primary amino groups of said polyamine at least in part to secondary amino groups of the formula:
- NH - CH₂ - CH₂ - COOR
wherein R is a n-butyl, isobutyl, t-butyl, or 2-ethylhexyl group.

2. The composition according to Claim 1, wherein at least 30 % of said primary amino groups are converted to said secondary amino groups.

3. The composition according to Claim 1 or 2, wherein said polyoxypropylene triamine has an average molecular weight from 200 to 10,000.

4. The use of a composition as defined in any one of claims 1 to 3 as a reactant in polyurea reaction injection molding (RIM).

## Patentansprüche

1. Zusammensetzung zur Verwendung bei der Herstellung von Kunststoffen, umfassend ein Polyaminprodukt, das erhältlich ist durch Umsetzung eines unter n-Butylacrylat, Isobutylacrylat, t-Butylacrylat und 2-Ethylhexylacrylat ausgewählten Acrylatesters mit einem unter Polyoxypropylentriamin, Tolylendiamin und Diethyltolylendiamin ausgewählten Polyamin, um die primären Aminogruppen des Polyamins wenigstens teilweise zu sekundären Aminogruppen der Formel:
-NH-CH₂-CH₂-COOR
umzuwandeln,
worin R für eine n-Butyl-, Isobutyl-, t-Butyl- oder 2-Ethylhexyl-Gruppe steht.

2. Zusammensetzung nach Anspruch 1, worin wenigstens 30 % der primären Aminogruppen zu sekundären Aminogruppen umgewandelt werden.

3. Zusammensetzung nach Anspruch 1 oder 2, worin das Polyoxypropylentriamin ein mittleres Molekulargewicht von 200 bis 10 000 besitzt.

4. Verwendung einer Zusammensetzung nach einem der Ansprüche 1 bis 3 als Reaktand für das Polyharnstoff-RIM.

## Revendications

1. Composition à utiliser dans la fabrication de plastiques comprenant un produit polyamine qu'on peut obtenir en faisant réagir un ester d'acrylate choisi parmi l'acrylate de n-butyle, l'acrylate d'isobutyle, l'acrylate de t-butyle et l'acrylate de 2-éthylhexyle, avec une polyamine choisie parmi une polyoxypropylène triamine, la tolylènediamine et la diéthyltolylènediamine, pour transformer les groupes amine primaire de ladite polyamine au moins en partie en groupes amine secondaire de formule :
- NH - CH₂ - CH₂ - COOR
dans laquelle R est un groupe n-butyle, isobutyle, t-butyle, ou 2-éthylhexyle.

2. Composition selon la revendication 1, dans laquelle au moins 30 % desdits groupes amine primaire sont transformés en lesdits groupes amine secondaire.

3. Composition selon la revendication 1 ou 2, dans laquelle ladite polyoxypropylène triamine a un poids moléculaire moyen de 200 à 10 000.

4. Utilisation de la composition selon l'une quelconque des revendications 1 à 3 comme réactif dans la R.M.I. de polyurée.
